# EUROPEAN PATENT APPLICATION

(11) **EP 2 676 602 A1**
(43) Date of publication of application: **25.12.2013**
(21) Application number: 12172741.6
(22) Date of filing: 20.06.2012
(51) Int. Cl.: A61B 5/0408, A61B 5/0492

(54) **Physiological signal detection device**

(71) Applicant: King's Metal Fiber Technologies Co., Ltd., Taichung City (TW)
(72) Inventor: Huang, Hong-Hsu, Taipei City (TW); Su, I-Chen, Taipei City (TW); Yang, Shun-Tung, Taipei City (TW)
(74) Representative: Viering, Jentschura & Partner

(57) **Abstract**

A physiological signal detection device (1) includes a base layer (20), at least one electrode pad (11), and at least one projection (30). The electrode pad is positioned on a top surface of the base layer. The electrode pad and the base layer form a first receiving compartment (S1) therebetween. The projection is retained in the first receiving compartment. The projection includes at least one raised surface (31), and the raised surface of the projection supports and raises the electrode pad.

## Description

### FIELD OF THE INVENTION

The present invention relates to a physiological signal detection device, and in particular to a physiological signal detection device that comprises a prominent projection for easy contact and secure engagement with a surface of human body, in order to facilitate detection of physiological signals of human body.

### BACKGROUND OF THE INVENTION

Nowadays, to detect physiological signals of human body, such as heart beat and brain wave, a plurality of sensor elements of physiological detection equipment is attached to (or worn on) various sites on a surface of human body. These sensor elements detect the current that spreads to the peripheral tissues or body surface occurring when nervous impulses (namely variation of membrane potential) passes through human body organs (such as heart and head). The detected signal is then transmitted from the sensor elements by electrical wires to the physiological detection equipment to be processed and converted into data to be displayed. In this way, the condition of an inspected portion (such as heart rate and variation of brain wave) can be realized.

The sensor elements are often made in the form of an electrode pad. The conventional electrode pad has a structure comprising an adhesive base layer (such as a layer of conductive adhesive) and an electrical conductive portion bonded to the base layer. To use in inspecting physiological signals, a surface of the base layer is completely and adhesively attached, through the adherence thereof, to the human body surface, whereby electrical current on the human body surface may flow through the base layer, the electrically conductive portion, electrical wires, to the physiological detection equipment. On the other hand, to carry out electrotherapy, electrical current is transmitted from the physiological detection equipment to the human body surface to penetrate into the body surface to simulate the portion to be treated.

However, the human body surface is a curved surface that is irregularly raised and recessed, particularly for prominently projecting portions (such as shoulders and knees), and this often leads to a gap existing between the base layer of the conventional electrode pad and the human body surface. When the human body makes movement, the peripheral portions of shoulder blade or the elbow joint may push away the base layer of the conventional electrode pad. This may lead to partial separation of the base layer and no longer attached to the human body surface, or even completely detached. These situations make it not possible to generate any detection signal and may lead to interruption of detection. Further, when a plurality of conventional electrode pads is used, these pads must be separately positioned on various portions of human body surface to be detected and then fixed. This makes the use very troublesome.

Further, the base layer of the conventional electrode pad may get cracking due to drying and may be easily stained with sweat dirt to have the transmission of physiological signal affected. Further, the retained sweat dirt may lead to moldiness and blackening. Or it may be easy to stick other contaminants (such as dusts) to induce noise interference. Further, the sweat dirt or contaminants that attach to the surface of the base layer may make the base layer losing adherence. Thus, the conventional electrode pad is often damaged due to the above discussed problems and inconvenience of use may result.

In view of these problems, the present invention aims to provide a physiological signal detection device that forms distinct projections and is easy to attach to and securely bond to human body surface in order to make inspection of physiological signals of an inspection subject easy and to increase convenience of use.

### SUMMARY OF THE INVENTION

An object of the present invention is to provide a physiological signal detection device that forms distinct projections and is easy to attach to and securely bond to human body surface in order to make inspection of physiological signals of an inspection subject easy and to increase convenience of use.

Another object of the present invention is to provide a physiological signal detection device that comprises an edge enclosure to provide the functions of positioning and improving comfortableness and aesthetics and also reduce the influence thereof to conduction of electrical current and also reduce noise interference.

To achieve the above objects, the present invention provides a physiological signal detection device that comprises a base layer; at least one electrode pad, which is positioned on a top surface of the base layer, the electrode pad and the base layer forming a first receiving compartment therebetween; and at least one projection, which is retained in the first receiving compartment. The projection comprises at least one raised surface, and the raised surface of the projection supports and raises the electrode pad. As such, a prominent projection is provided for easy contact and secure engagement with the surface of human body in order to facilitate detection of physiological signal of an inspection subject and improve convenience of operation. Further, an edge enclosure band is also included to provide function of positioning, improving comfortableness and aesthetics, and reducing influence on conduction of electrical current and lowering noise interference.

### BRIEF DESCRIPTION OF THE DRAWINGS

The present invention will be apparent to those skilled in the art by reading the following description of preferred embodiments thereof with reference to the drawings, in which:

Figure 1 is a perspective view showing a physiological signal detection device according to the present invention;

Figure 2 is a cross-sectional view taken along line A-A of Figure 1;

Figure 3 is a cross-sectional view, in an enlarged form, taken along line B-B of Figure 1;

Figure 4 is an exploded view of Figure 1;

Figure 5 is a schematic view showing the physiological signal detection device of Figure 2 with an edge enclosure band, sewing threads, and an extension conductor removed;

Figure 6 is a schematic view showing the physiological signal detection device of Figure 2 with a connection band and extension conductors removed;

Figure 7 is a schematic view showing a simple structure of the physiological signal detection device according to the present invention;

Figure 8 is an exploded view of the physiological signal detection device shown in Figure 7 further comprising an edge enclosure band;

Figure 9 is a perspective view showing the physiological signal detection device of Figure 8 after being assembled;

Figure 10 is rear view showing the physiological signal detection device according to the present invention coupled to a back of a garment; and

Figure 11 is a front view showing the physiological signal detection device according to the present invention coupled to a front-open garment.

### DETAILED DESCRIPTION OF THE PREFERRED EMBODIMENTS

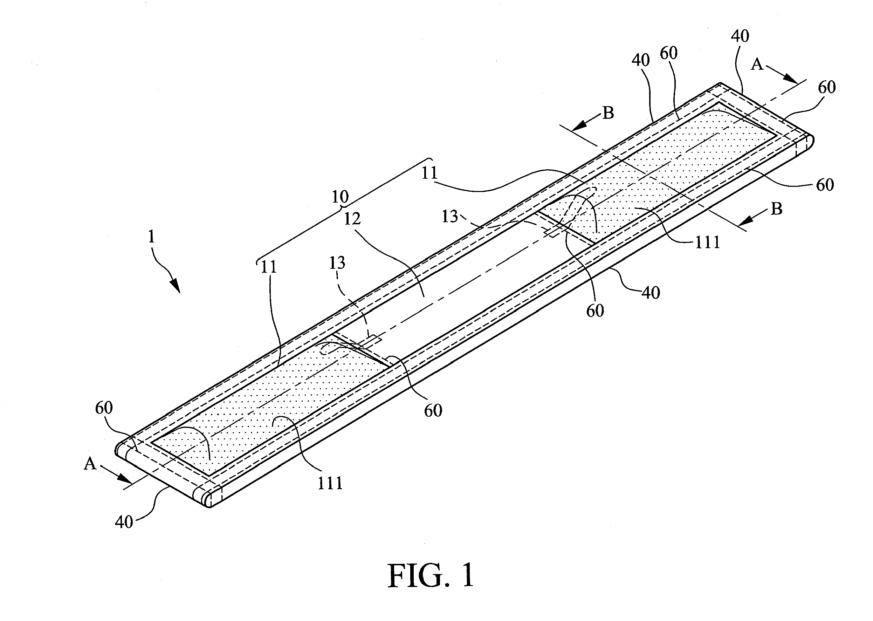

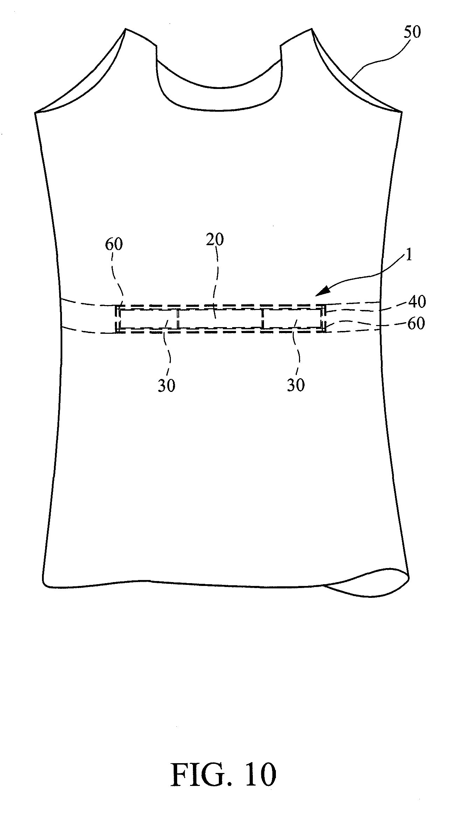

With reference to the drawings and in particular to Figures 1-9, a physiological signal detection device constructed in accordance with the present invention, generally designated at 1, comprises a combination of at least one electrode pad 11, a base layer 20, and at least one projection 30. The electrode pad 11 functions to set in contact with a surface of human body. The projection 30 is arranged to correspond to the electrode pad 11. The base layer 20 is directly attachable to or coupleable to a wearable article (such as garment 50 of Figure 10).

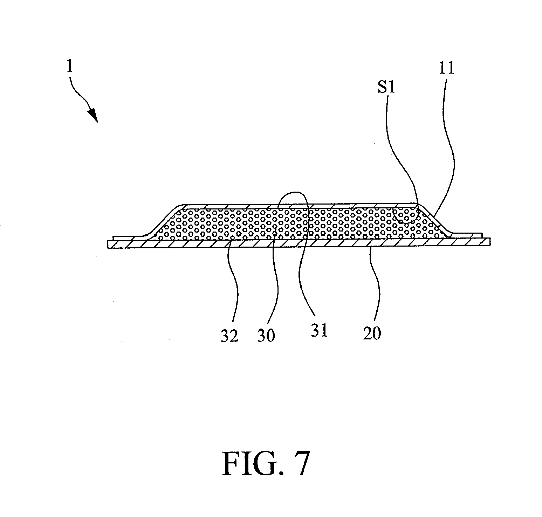

In an example way of practice, the physiological signal detection device 1 has a simple structure of which the assembled configuration is illustrated in Figure 7, which is embodied in the form of a single electrode pad 11. The electrode pad 11 is positioned on a top surface of the base layer 20 in such a way that the electrode pad 11 and the base layer 20 form therebetween a first receiving compartment S1. The projection 30 is received and fixed in the first receiving compartment S1. The projection 30 has at least one raised surface 31, and the raised surface 31 of the projection 30 supports and raises the electrode pad 11.

With the above discussed constituent components, the present invention, when attached to the surface of human body, may be set in compliance with the surface curve of the human body to tightly attach thereto. The projection 30 is set in alignment with and supports and raises the electrode pad 11 so as to make a face of the electrode pad 11 projecting upward, whereby when observed from outside, the local face of the electrode pad 11 shows a projecting configuration to help tightly engage (or tightly abut against) the surface of human body with substantially no gap existing therebetween to allow electrical current on the surface of human body to be easily transmitted to the electrode pad 11 and facilitating detection of variation of physiological signal.

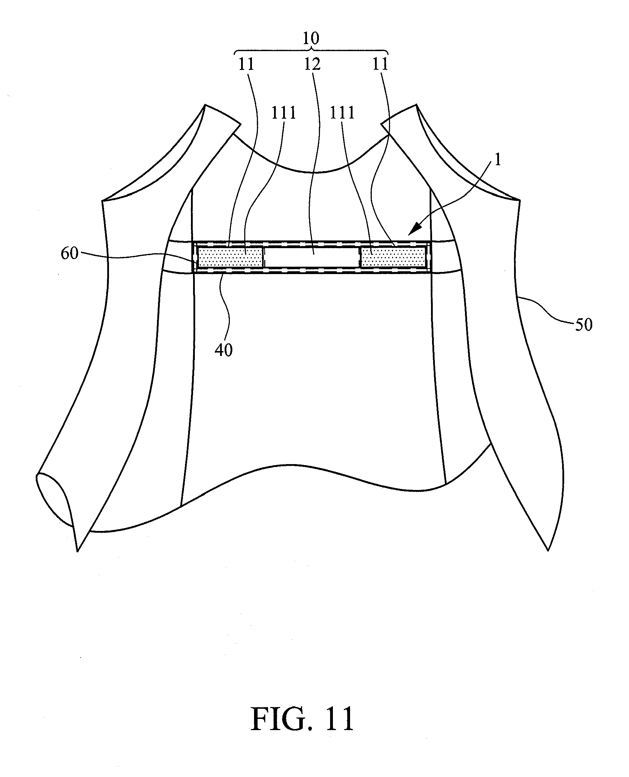

The physiological signal detection device 1 can be embodied in various formed according different number of electrode pads 11 used. For an embodiment in which the present invention is embodied with a single electrode pad 11, a wearable article (such as a wrist band or a wrist protector, not shown) to which the physiological signal detection device 1 according to the present invention is combined, together with an electronic device (such as a multimedia playing device, not shown), with an opposite end of the electronic device being electrically connected to an accessory (such as an earphone, not shown) that is attached to another portion of human body (such as an ear) for grounding (or negative electric pole) purposes so as to form a detection circuit. For an embodiment in which the present invention is embodied with a plurality of electrode pads 11, the physiological signal detection device 1 of the present invention is not limited to being mounted to wrist and may, as shown in Figure 11, be coupled to a garment 50 in such a way that two electrode pads 11 that are mounted inside the garment 50 form a detection circuit.

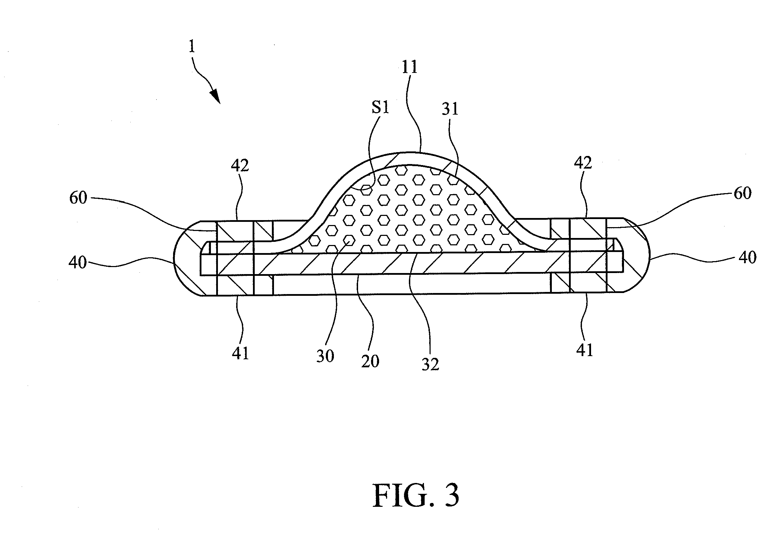

Opposite to the raised surface 31, the projection 30 comprises an opposite surface 32, which can be embodied in a raised form (not shown) or a flat form (see Figure 3). Illustratively, the opposite surface 32 is embodied in a flat form in such a way that the overall configuration of the projection 30 is converging from the opposite surface 32 toward the raised surface 31, so that besides contact area between the opposite surface 32 of the projection 30 and the base layer 20 being increased, contact area between the raised surface 31 of the projection 30 and the electrode pad 11 is also increased to ensure a tight engagement. In this way, the projection 30 is securely positioned in the first receiving compartment S1 against sliding and shaking and the raised surface 31 is made in alignment with and supporting the electrode pad 11 is a projecting manner, reducing gap between the raised surface 31 and the electrode pad 11.

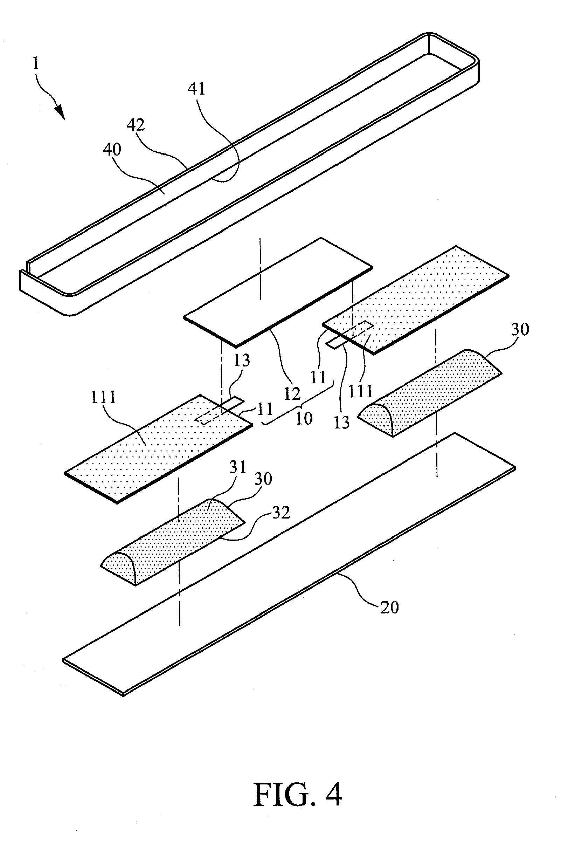

The electrode pad 11 is provided with a plurality of mesh pores 111 (see Figures 1 and 4) to block foreign objects, such as dusts, from penetrating into the electrode pad 11 so as to reduce interference with conductive portions inside the electrode pad 11 and minimizes generation of noise.

The projection 30 can be embodied with foam, silicone rubber, cotton fabric, or elastic objects for easy manufacture. For an embodiment where the projection 30 is made of an elastic object, the projection shows resiliency and is expandable or compressible and is prone to bending and folding and may resume the original shape thereafter so as to easily comply with the curve of surface of human body when attached to the surface of human body thereby facilitating easy use and replacement.

The base layer 20 may comprise a fabric layer, a plastic sheet, or a layer of polyester fibers.

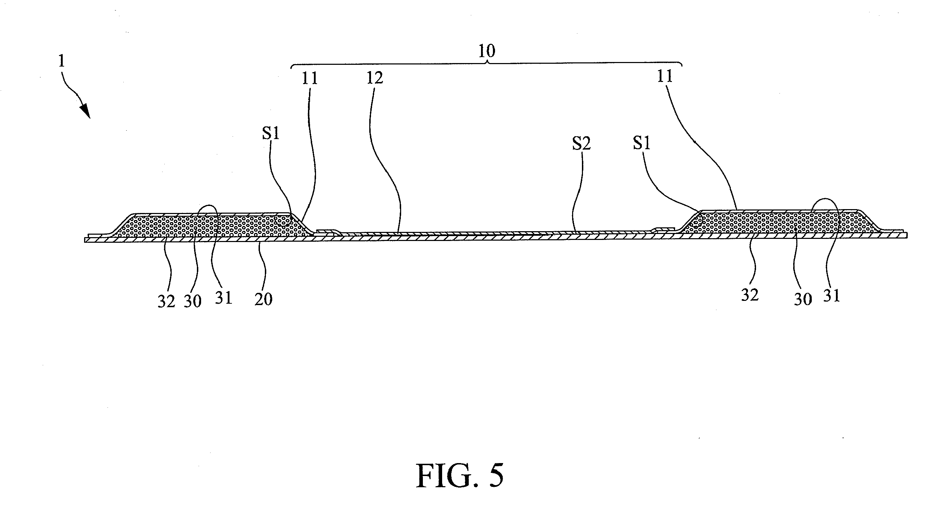

Further, in the instant embodiment, the at least one electrode pad 11 can be a plurality of electrode pads 11, and the at least one projection 30 can be a plurality of projections 30. The term "a plurality of" refers to a number that is equal to or greater than two. An example of the present invention shown in Figure 5 (in combination with Figures 1, 2, and 4) comprises two electrode pads 11 used in combination with two projections 30. The two electrode pads 11 are arranged, in a mutually spaced manner, on the top surface of the base layer 20 in such a way that each of the electrode pads 11 forms a first receiving compartment S1 with respect to the base layer 20 and each of the projections 30 is received and retained in each of the first receiving compartments S1. Each of the projections 30 has at least one raised surface 31, and the raised surface 31 of each of the projections 30 supports and raises each of the electrode pads 11. Further, at least one connection band 12 is connected between any two adjacent ones of the electrode pads 11 in such a way that opposite ends of the connection band 12 are respectively coupled to the two adjacent electrode pads 11, whereby a second receiving compartment S2 is formed between the connection band 12 and the base layer 20. The electrode pads 11 and the connection band 12 are jointed together to form the top layer 10 that corresponds in shape to the base layer 20. In this way, being assisted by the connection band 12, the electrode pads 11 can efficiently attached to opposite sides of a portion of human body (such as left and right sides of a head, left and right side parts of a back, or opposite portions of chest and back of a heart-associated portion).

The second receiving compartment S2 functions to receive and retain other objects (such as electrical wires, sensors, and controllers). In case that no article or object is received in the second receiving compartment S2, the connection band 12 is bonded to the top surface of the base layer 20.

In a practical application, connection made between each of the electrode pads 11 and the base layer 20 and the connection band 12, and connection made between the connection band 12 and the base layer 20 include sewing (being stitched with threads), adhesives (being bonded with application of adhesives) or thermal fusion (being first cut and heating applied to the cut site for fusion and jointing) to ensure secure connection.

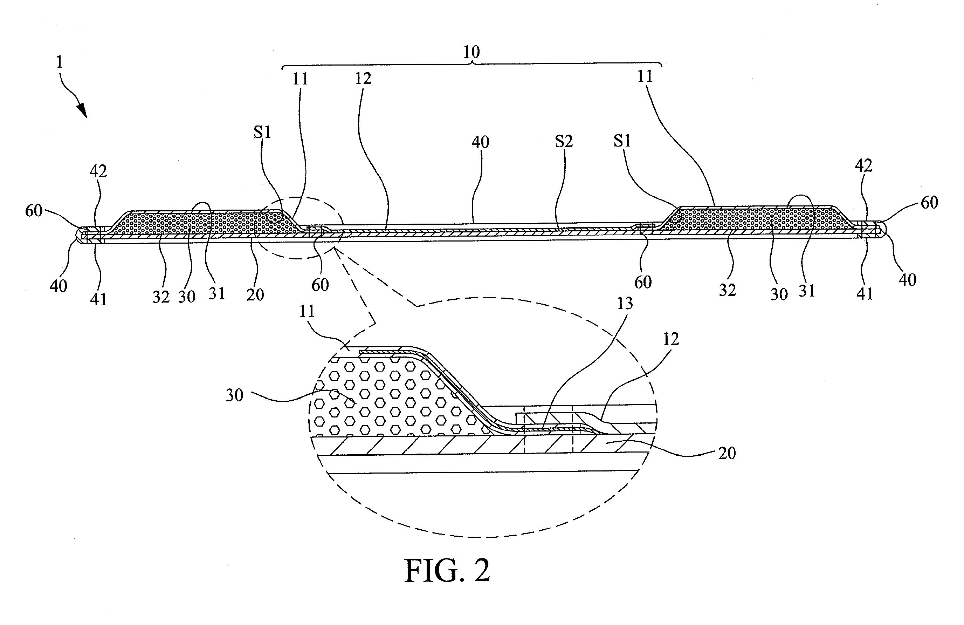

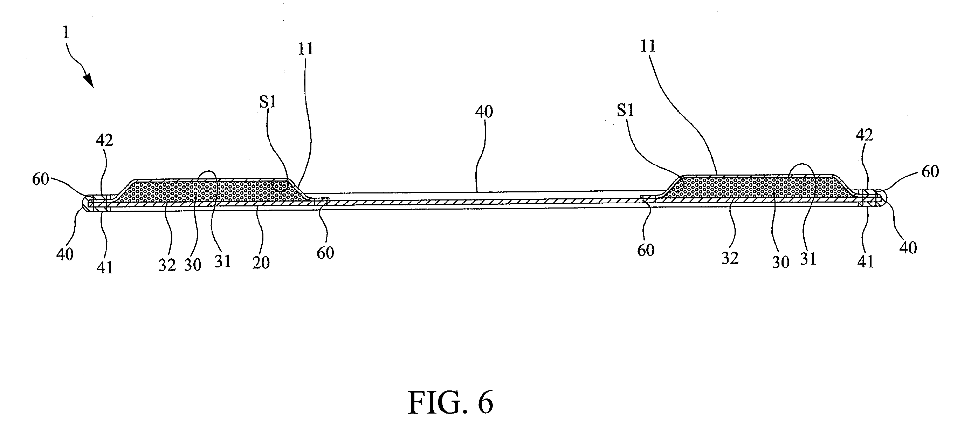

Further, as shown in Figures 1-4, in a preferred embodiment, the physiological signal detection device 1 further comprises an edge enclosure band 40, of which the structure of embodiment is shown in Figures 2 and 3. The edge enclosure band 40 has a lower flange 41 that is coupled to an edge portion of a bottom surface of the base layer 20 and the edge enclosure band 40 also has an upper flange 42 that is coupled to an edge portion of a top surface of the top layer 10 (which is composed of the connected electrode pads 11 and connection band 12). The edge portion of the top surface of the top layer 10 corresponds to the edge portion of the bottom surface of the base layer 20 so as to allow the edge enclosure band 40 to effect enclosure and retention for improving comfortableness and aesthetics. In the instant embodiment, the connection band 12 is optionally provided based on the practical needs. Thus, the present invention can be embodied as shown in Figure 6 to arrange two mutually-spaced electrode pads 11 on a top surface of the base layer 20 with the upper flange 42 of the edge enclosure band 40 being only coupled to the edge portions of top surfaces of the two electrode pads 11. In this way, when the present invention is attached to a garment 50 (see Figures 10 an 11) by sewing, sewn area can be easily identified with the assistance of the edge enclosure band 40, whereby sewing thread 60 is applied along the edge enclosure band 40 to prevent mistakenly penetrating through the projection and thus damaging the structure. The present invention is applicable in combination with a garment 50, which, when worn by an inspection subject, allows the two electrode pads 11 to be quickly set corresponding to and conveniently attached, in a tight engagement, to the portion to be inspected, eliminating the need for separately attaching the pads.

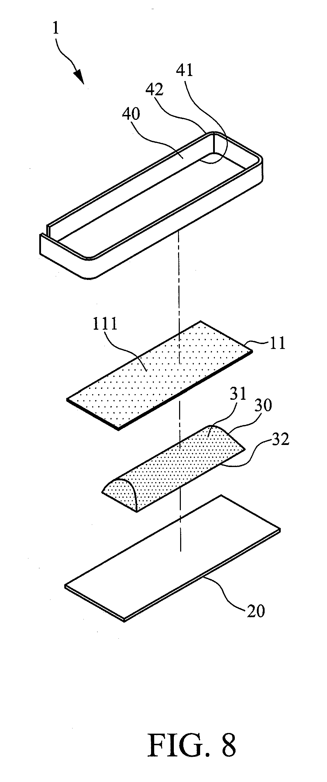

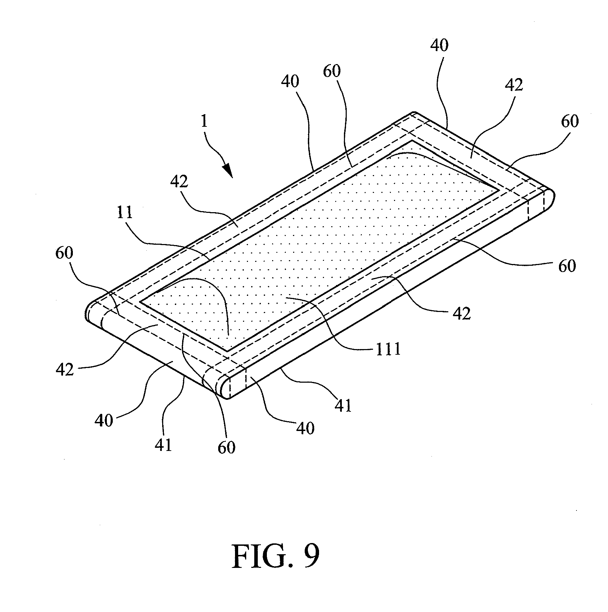

Further, as shown in Figures 8 and 9, the edge enclosure band 40 can be embodied in combination with the previously discussed simple structure embodiment of the physiological signal detection device 1 (such a simple structure embodiment being shown in Figure 7 and a combined embodiment being shown in Figure 9) in such a way that the lower flange 41 of the edge enclosure band 40 is coupled to an edge portion of a bottom surface of the base layer 20, and the upper flange 42 of the edge enclosure band 40 is coupled to an edge portion of a top surface of the electrode pad 11, in which the edge portion of the top surface of the electrode pad 11 corresponds to the edge portion of the bottom surface of the base layer 20 in order to facilitate enclosure and retention effected by the edge enclosure band 40 and to improve comfortableness and aesthetics and help attaching to the garment 50.

Further, for an electrode pad 11 of a relatively large size, to save expense and to effectively form electrical engagement between the electrode pad 11 and body surface, as shown in Figures 1, 2, and 4, each of the electrode pads 11 is provided with at least one extension conductor 13 (which is particularly shown in the enlarged view at left lower corner of Figure 2) connected thereto. The extension conductor 13 has an end extending into and electrically connected to the electrode pad 11 and the extension conductor 13 has an opposite end projecting beyond an end of the electrode pad 11, whereby easy electrical connection of the extension conductor 13 of the electrode pad 11 can be made with electrical current of the surface of human body when the electrode pad 11 is supported and raised by the projection 30. In a practical application, where the present invention is embodied with a single electrode pad 11, the extension conductor 13 discussed above can also be included. Further, when the present invention is embodied with more than two electrode pads 11, based on the sizes thereof, the electrode pads 11 can be selectively provided with and coupled to extension conductors 13.

The extension conductor 13 discussed above for the electrode pad 11 is formed by composing a plurality of conductive yarns that are provided for weaving purposes and are relatively flexible. The extension conductor 13 is electrically connected to an electrical wire (not shown), which has an opposite end electrically connected to physiological detection equipment (not shown), whereby electrical current of body surface flows through the electrode pad 11, the extension conductor 13, and the electrical wire to enter the physiological detection equipment. In a practical application, the extension conductor 13 is not a necessary component and the electrode pad 11 is directly and electrically connected to the electrical wire (not shown).

As such, the present invention provides a physiological signal detection device, which comprises a combined structure of at least one electrode pad 11, a base layer 20, and at least one projection 30 to provide a prominent projection for easy contact with and secure engagement with a surface of human body in order to facilitate detection of physiological signal of an inspection subject and improve convenience of operation. Further, an edge enclosure band 40 is also included to provide function of positioning, improving comfortableness and aesthetics, and reducing influence on conduction of electrical current and lowering noise interference.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A physiological signal detection device, comprising:
a base layer;
at least one electrode pad, which is positioned on a top surface of the
base layer, the electrode pad and the base layer forming a first receiving compartment therebetween; and
at least one projection, which is retained in the first receiving compartment,
the projection comprising at least one raised surface, the raised surface of the projection supporting and raising the electrode pad.

2. The physiological signal detection device as claimed in Claim 1, wherein the electrode pad is provided with a plurality of mesh pores.

3. The physiological signal detection device as claimed in Claim 1, wherein the projection comprises one of foam, silicone rubber, and cotton fabric.

4. The physiological signal detection device as claimed in Claim 1, wherein the projection comprises an elastic object.

5. The physiological signal detection device as claimed in Claim 1, wherein the base layer comprises one of fabric layer, plastic sheet, and a layer of polyester fibers.

6. The physiological signal detection device as claimed in Claim 1, wherein the at least one electrode pad includes a plurality of electrode pads, which are arranged, in a mutually spaced manner, on the top surface of the base layer, each of the electrode pads and the base layer forming therebetween a first receiving compartment, the at least one projection including a plurality of projections, each of the projections being received and retained in each of the first receiving compartments, each of the projections having at least one raised surface, the raised surface of each of the projections supporting and raising each of the electrode pads, a connection band being connected to two adjacent ones of the electrode pads, the connection band and the base layer forming therebetween a second receiving compartment, the electrode pads and the connection band being connected to each other to form a top player corresponding in shape to the base layer.

7. The physiological signal detection device as claimed in Claim 6 further comprising an edge enclosure band, which has a lower flange coupled to an edge portion of the bottom surface of the base layer and an upper flange coupled to an edge portion of the top surface of the top layer, the edge portion of the top surface of the top layer corresponding to an edge portion of a bottom surface of the base layer.

8. The physiological signal detection device as claimed in Claim 6, wherein each of the electrode pads is further provided with at least one extension conductor, which has an end extending into and electrically with each of the electrode pads and an opposite end extending beyond one side of each of the electrode pads.

9. The physiological signal detection device as claimed in Claim 1 further comprising an edge enclosure band, the edge enclosure band having a lower flange coupled to an edge portion of a bottom surface of the base layer and an upper flange coupled to an edge portion of a top surface of the electrode pad, the edge portion of the top surface of the electrode pad corresponding to the edge portion of the bottom surface of the base layer.
